# EUROPEAN PATENT APPLICATION

(11) **EP 4 215 231 A1**
(43) Date of publication of application: **26.07.2023**
(21) Application number: 21868101.3
(22) Date of filing: 26.04.2021
(51) Int. Cl.: A61M 5/32

(54) **SAFETY DEVICE FOR PRE-FILLED SYRINGE AND INJECTION DEVICE**

(30) Priority: 18.09.2020 CN 202022070313 U
(71) Applicant: Shandong Wego Prefills Pharmaceutical Packaging Co., Ltd., Weihai, Shandong 264210 (CN)
(72) Inventor: SUN, Xiaofeng, Weihai, Shandong 264210 (CN); XU, Qianhe, Weihai, Shandong 264210 (CN); PENG, Tongyi, Weihai, Shandong 264210 (CN); LI, Wenzheng, Weihai, Shandong 264210 (CN); LI, Zhifei, Weihai, Shandong 264210 (CN)
(74) Representative: Ungria López, Javier
(86) International application number: PCT/CN2021/089864
(87) International publication number: WO 2022/057263

(57) **Abstract**

A safety device for a pre-filled syringe and an injection device; the inner wall of a device body (5) of the safety device is provided with a snap-fitting portion (10) and a bearing portion (5.1); a protective cover (6) is movably provided in the device body (5), and the protective cover (6) is provided with a hook claw (6.1) capable of being fitted with the snap-fitting portion (10) for positioning and a positioning base (6.4) for limiting the protective cover (6) from being separated from the device body (5); in a state where a needle tip (11) is covered by the protective cover (6), the hook claw (6.1) is separated from the snap-fitting portion (10), and the positioning base (6.4) is in contact with the bearing portion (5.1); a release ring (8) is movably provided in the device body (5), one end of the release ring (8) towards the protective cover (6) can separate the hook claw (6.1) from the snap-fitting portion (10) by pressing the hook claw (6.1), and the other end of the release ring (8) away from the protective cover (6) is a positioning surface used for being positioned in contact with a syringe flange (13.1); two ends of an elastic component (7) abut against the release ring (8) and the protective cover (6) respectively; and a stent (9) is connected to the device body (5), and the stent (9) is provided with an inner stent claw (9.2) for limiting the syringe flange (13.1) to move in the direction away from the release ring (8). The safety device does not interfere with the injection operation of the pre-filled syringe, and prevents the protective cover from popping up in advance.

## Description

This application claims priority to Chinese Patent Application No. 202022070313.8, titled "SAFETY DEVICE FOR PRE-FILLED SYRINGE AND INJECTION DEVICE", filed on September 18, 2020 with the China National Intellectual Property Administration, which is incorporated herein by reference in its entirety.

### FIELD

The present application relates to the technical field of medical devices and in particular to a safety device for a pre-filled syringe and an injection device.

### BACKGROUND

A pre-filled syringe for medical use includes a cylinder having a needle for hypodermic injection at one end and a plunger configured to be movable within the cylinder, such that an inward stroke of the plunger causes a substance contained within the cylinder to be expelled from a needle hole.

A safe syringe typically includes some form of safety mechanism to protect medical staffs from injury from the needle for hypodermic injection after the needle for hypodermic injection has injected the substance into a patient. A typical safe syringe may include a protective cover for covering the needle or may allow the needle to be retracted into the cylinder of the syringe.

In the conventional technology, to avoid a risk of an accidental needle pricking, many hypodermic syringes are provided with a rigid cylindrical protective cover retractable along the cylinder of the syringe. The protective cover is movable between a retracted position where the needle is exposed for use and an extended position where the needle is surrounded by the protective cover.

Due to a risk of infectious diseases, a number of syringes and related safety devices have been developed to prevent an accidental needle pricking and/or unintentional reuse of the syringe, thereby achieving a safety protection. A conventional passive anti-needle pricking safety device for a pre-filled syringe is required to be installed to the syringe. The safety device is not allowed to interfere excessively with a force required to move a plunger rod during the injection or hinder an entire stroke of the plunger rod, thereby not interfering with an injection operation. It is necessary to ensure that the safety device is triggered only after the drug injection has been fully completed (that is, the plunger rod is near an end of the stroke of the plunger rod), so as to avoid premature ejection of the protective cover.

Therefore, how to not interfere with the injection operation and to avoid premature ejection of the protective cover has become an issue to be urgently solved by those skilled in the art.

### SUMMARY

It is an object of the present application to provide a safety device for a pre-filled syringe that does not interfere with an injection operation and avoids premature ejection of a protective cover. An injection device having the same is further provided according to the present application.

To solve the above technical problem, a safety device for a pre-filled syringe is provided according to the present application. The safety device includes a device body, a protective cover, a release ring, an elastic member, and a holder.

An inner wall of the device body is provided with a snap-fitting portion and a bearing portion.

The protective cover is movably arranged in the device body, where the protective cover is provided with a claw capable of fitting with the snap-fitting portion for positioning and a positioning seat configured to restrain the protective cover from escaping from the device body, where in a state that a needle tip of the pre-filled syringe is covered by the protective cover, the claw is detached from the snap-fitting portion and the positioning seat is in contact with the bearing portion.

The release ring is movably arranged in the device body, where an end, facing the protective cover, of the release ring is capable of detaching the claw from the snap-fitting portion by squeezing the claw, and another end, away from the protective cover, of the release ring is a positioning surface used for contacting with a syringe flange of the pre-filled syringe for positioning.

An end of the elastic member abuts against the release ring and another end of the elastic member abuts against the protective cover.

The holder is connected with the device body, where the holder is provided with a holder inner claw for restraining the syringe flange from moving away from the release ring.

In an embodiment, in the safety device for a pre-filled syringe, an outer wall of the device body is provided with an external flange.

In an embodiment, in the safety device for a pre-filled syringe, the holder inner claw is arranged at an end, away from the protective cover, of the holder; a holder outer claw is arranged at another end, closer to the protective cover, of the holder, the device body is provided with an opening portion, and the holder outer claw is fitted into the opening portion for connection.

In an embodiment, in the safety device for a pre-filled syringe, a top projection ring is provided at an end, away from the protective cover, of the release ring, and the positioning surface is a top surface of the top projection ring; an inner end face of the top projection ring extends into an inner bore of the release ring, and an end of the elastic member extends into the inner bore of the release ring and abuts against the inner end face.

In an embodiment, in the safety device for a pre-filled syringe, an end, facing the protective cover, of the release ring is a squeezing end for squeezing the claw.

In an embodiment, in the safety device for a pre-filled syringe, the holder has a hollow structure.

In an embodiment, in the safety device for a pre-filled syringe, the bearing portion is of a protruding ring structure with a first end face facing the release ring and a second end face away from the release ring; a face, away from the release ring, of the positioning seat is a limiting face; and in the state that the needle tip of the pre-filled syringe is covered by the protective cover, the limiting face is in contact with the first end face for positioning.

In an embodiment, in the safety device for a pre-filled syringe, the protective cover is further provided with a positioning elastic sheet; and in the state that the needle tip of the pre-filled syringe is covered by the protective cover, the positioning elastic sheet is in contact with the second end face.

In an embodiment, in the safety device for a pre-filled syringe, the elastic member is a spring.

An injection device is further provided according to the present application. The injection device includes a pre-filled syringe and the safety device according to any one of the above aspects.

With the safety device for a pre-filled syringe according to the present application, a syringe body of the pre-filled syringe may be sequentially directed through the release ring, the elastic member and the protective cover, with the syringe body being arranged in the device body. The connection of the holder to the device body allows the holder inner claw to restrain the syringe flange from moving away from the release ring, so that the safety device is assembled to the pre-filled syringe. During the injection process, a push rod of the pre-filled syringe is pushed (a pushing force is an injection pressure). Since the syringe flange is in contact with and positioned to the positioning surface at an end, facing the protective cover, of the release ring, the syringe body is static relative to the device body. The push rod drives a plunger of the pre-filled syringe to move inside the syringe body and to compress the drug inside the syringe body towards the needle tip of the pre-filled syringe, so that the drug inside the syringe body is injected into a patient. After the injection, the plunger is in contact with and positioned to an end portion (an end, closer to the needle tip, of the syringe body) of the syringe body. When the plunger is no longer allowed to advance further relative to the needle tip, it indicates that the drug in the syringe body has been completely expelled, and the user then withdraws the needle tip from an injection site. The user then simply again increases the pressure (which is greater than the injection pressure) on the push rod after the needle tip has been withdrawn, so that the release ring and the protective cover approach each other against an elastic recovery force of the elastic member. That is, the syringe flange pushes the release ring to approach the protective cover, so that the end, facing the protective cover, of the release ring squeezes the claw of the needle protective cover, causing the claw to be detached from the snap-fitting portion. Moreover, under the elastic recovery force of the elastic member, the protective cover moves away from the release ring, so that the protective cover covers the needle tip of the pre-filled syringe. In addition, due to the contact between the positioning seat and the bearing portion, the protective cover is restrained from escaping from the device body. By means of the above configuration, the safety device for a pre-filled syringe does not interfere with the injection operation of the pre-filled syringe. The pre-filled syringe can be a common syringe without structural changes. Therefore, the safety device is highly versatile. Moreover, the claw is fitted with and positioned to the snap-fitting portion, which ensures the safe positioning of the protective cover within the device body during the injection operation. Only after the drug injection has been completely completed, the end, facing the protective cover, of the release ring squeezes the claw so that the claw is detached from the snap-fitting portion, thereby enabling the protective cover to be ejected relative to the device body. The protective cover can be released and the safety device can be triggered without additional action, which is simple to operate and effectively prevents the protective cover from ejecting prematurely.

### BRIEF DESCRIPTION OF THE DRAWINGS

FIG. 1 shows a schematic structural diagram of an injection device according to the present application;
FIG. 2 shows a schematic perspective view of the injection device according to the present application;
FIG. 3 shows a schematic perspective view of the injection device according to the present application, where a protective cover is already released;
FIG. 4 shows a schematic structural view of a pre-filled syringe according to the present application;
FIG. 5 shows a schematic exploded view of the pre-filled syringe and a safety device according to the present application;
FIG. 6 shows a schematic perspective view of the safety device according to the present application;
FIG. 7 shows a first schematic exploded view of the safety device according to the present application;
FIG. 8 shows a second schematic exploded view of the safety device according to the present application;
FIG. 9 shows a schematic partial view showing the assembly of the safety device with the pre-filled syringe according to the present application;
FIG. 10 shows a first schematic structural diagram of the safety device according to the present application, where the protective cover is not released;
FIG. 11 shows a second schematic structural diagram of the safety device according to the present application, where the protective cover is not released; and
FIG. 12 shows a schematic structural diagram of the safety device according to the present application, where the protective cover is released.

### DETAILED DESCRIPTION OF THE EMBODIMENTS

The core of the present application is to provide a safety device for a pre-filled syringe that does not interfere with an injection operation and avoids premature ejection of a protective cover. An injection device having the same is further provided according to the present application.

In order to provide those skilled in the art with a better understanding of technical solutions of the present application, the present application is described in further detail below in conjunction with the accompanying drawings and specific embodiments.

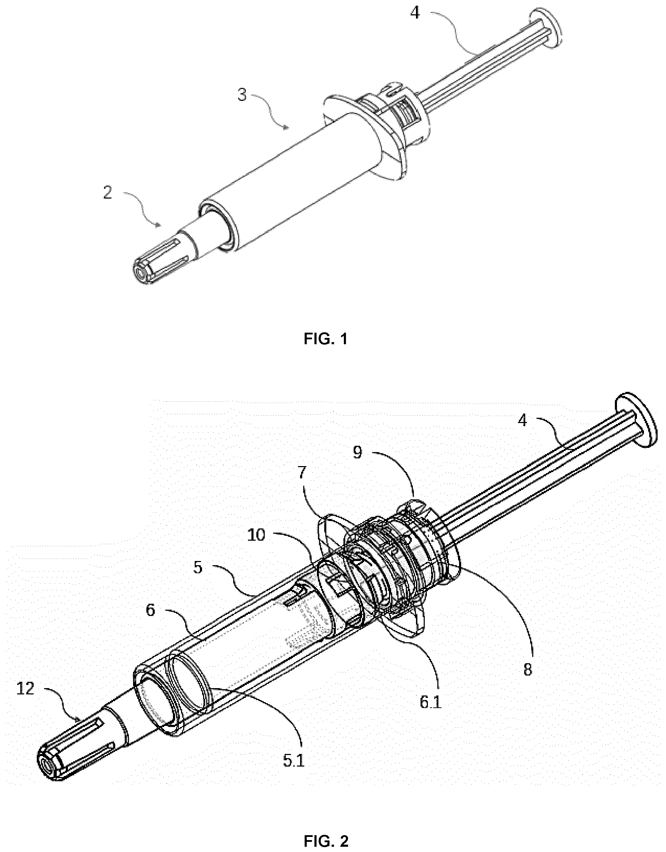

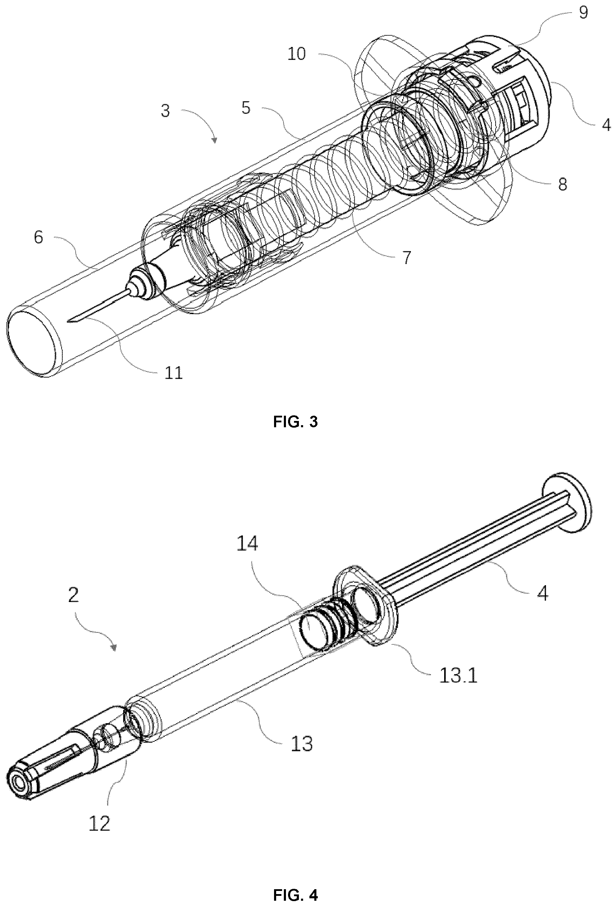

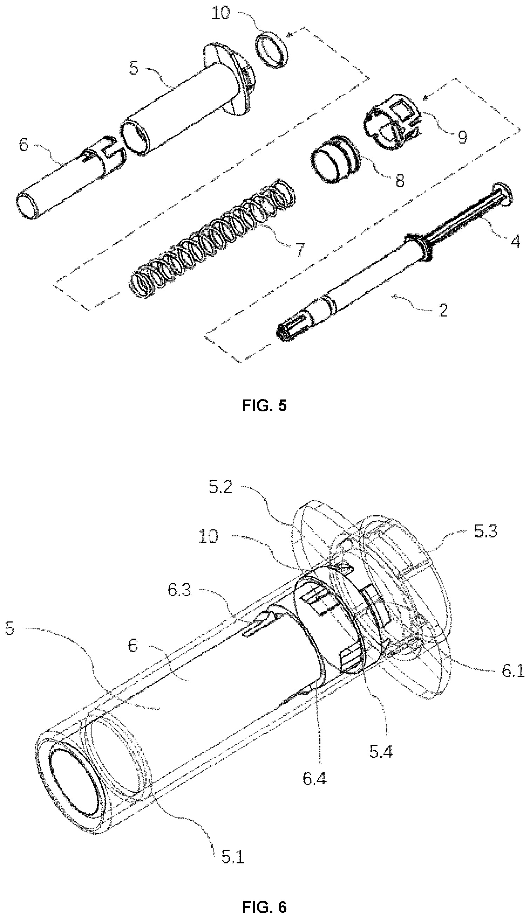

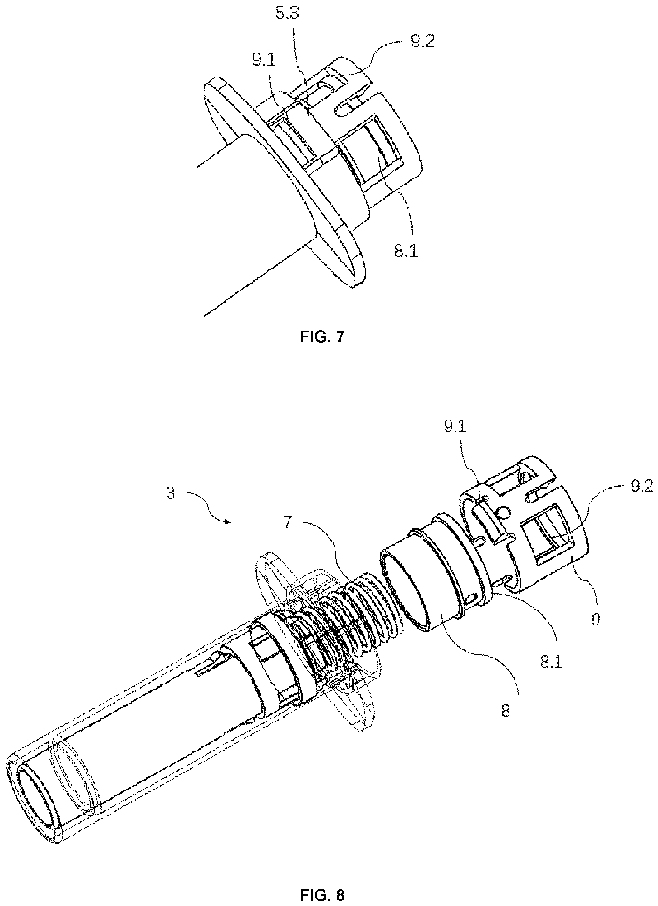

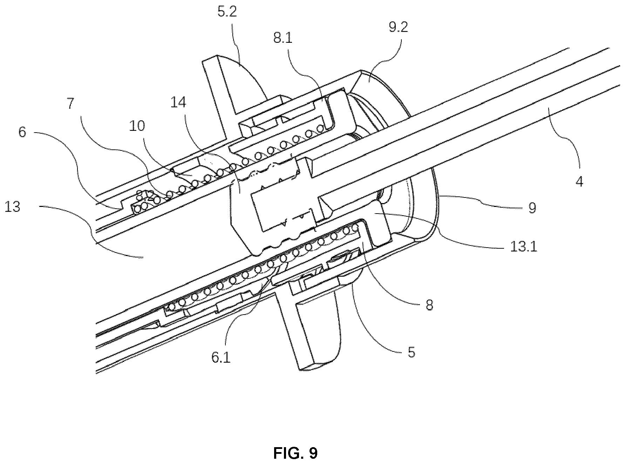

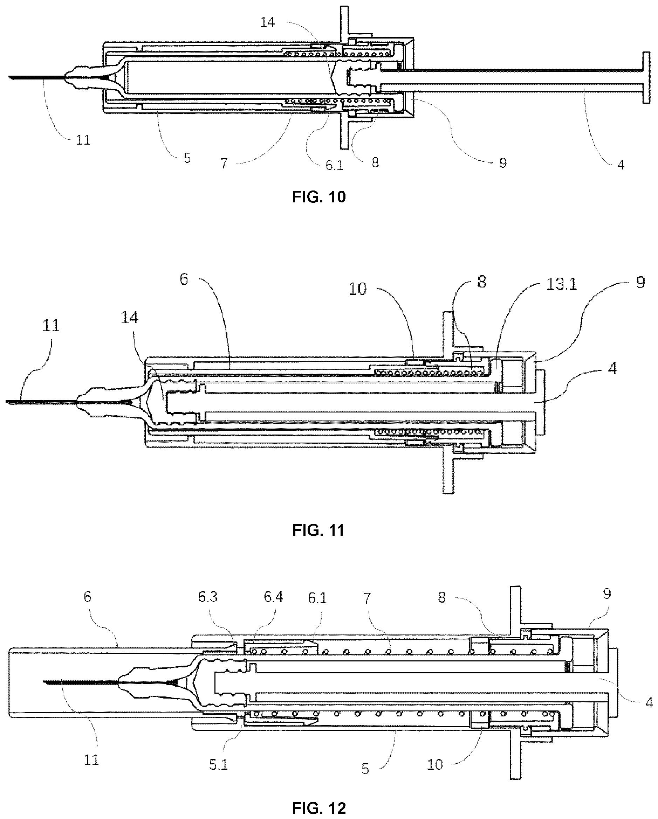

As shown in FIGS. 1 to 12, a safety device for a pre-filled syringe is provided according to an embodiment of the present application. The safety device includes a device body 5, a protective cover 6, a release ring 8, an elastic member 7 and a holder 9. An inner wall of the device body 5 is provided with a snap-fitting portion 10 and a bearing portion 5.1. The protective cover 6 is movably arranged in the device body 5. The protective cover 6 is provided with a claw 6.1 capable of fitting with the snap-fitting portion 10 for positioning and a positioning seat 6.4 configured to restrain the protective cover 6 from escaping from the device body 5. In a state that a needle tip 11 of a pre-filled syringe 2 is covered by the protective cover 6, the claw 6.1 is detached from the snap-fitting portion 10 and the positioning seat 6.4 is in contact with the bearing portion 5.1. The release ring 8 is movably arranged in the device body 5. An end, facing the protective cover 6, of the release ring 8 is capable of detaching the claw 6.1 from the snap-fitting portion 10 by squeezing the claw 6.1, and another end, away from the protective cover 6, of the release ring 8 is a positioning surface used for contacting with a syringe flange 13.1 of the pre-filled syringe 2 for positioning. An end of the elastic member 7 abuts against the release ring 8 and another end of the elastic member 7 abuts against the protective cover 6. The holder 9 is connected with the device body 5. The holder 9 is provided with a holder inner claw 9.2 for restraining the syringe flange 13.1 from moving away from the release ring 8.

With the safety device for a pre-filled syringe according to the embodiment of the present application, a syringe body 13 of the pre-filled syringe 2 may be sequentially directed through the release ring 8, the elastic member 7 and the protective cover 6, with the syringe body 13 being arranged in the device body 5. The connection of the holder 9 to the device body 5 allows the holder inner claw 9.2 to restrain the syringe flange 13.1 from moving away from the release ring 8, so that the safety device is assembled to the pre-filled syringe 2. During the injection process, a push rod 4 of the pre-filled syringe 2 is pushed (a pushing force is an injection pressure). Since the syringe flange 13.1 is in contact with and positioned to the positioning surface at an end, facing the protective cover 6, of the release ring 8, the syringe body 13 is static relative to the device body 5. The push rod 4 drives a plunger 14 of the pre-filled syringe 2 to move inside the syringe body 13 and to compress the drug inside the syringe body 13 towards the needle tip 11 of the pre-filled syringe 2, so that the drug inside the syringe body 13 is injected into a patient. After the injection, the plunger 14 is in contact with and positioned to an end portion (an end, closer to the needle tip 11, of the syringe body 13) of the syringe body 13. When the plunger 14 is no longer allowed to advance further relative to the needle tip 11, it indicates that the drug in the syringe body 13 has been completely expelled, and the user then withdraws the needle tip 11 from an injection site. The user then simply again increases the pressure (which is greater than the injection pressure) on the push rod 4 after the needle tip 11 has been withdrawn, so that the release ring 8 and the protective cover 6 approach each other against an elastic recovery force of the elastic member 7. That is, the syringe flange 13.1 pushes the release ring 8 to approach the protective cover 6, so that the end, facing the protective cover 6, of the release ring 8 squeezes the claw 6.1 of the needle protective cover, causing the claw 6.1 to be detached from the snap-fitting portion 10. Moreover, under the elastic recovery force of the elastic member 7, the protective cover 6 moves away from the release ring 8, so that the protective cover 6 covers the needle tip 11 of the pre-filled syringe 2. In addition, due to the contact between the positioning seat 6.4 and the bearing portion 5.1, the protective cover 6 is restrained from escaping from the device body 5. By means of the above configuration, the safety device for a pre-filled syringe does not interfere with the injection operation of the pre-filled syringe 2. The pre-filled syringe 2 can be a common syringe without structural changes. Therefore, the safety device is highly versatile. Moreover, the claw 6.1 is fitted with and positioned to the snap-fitting portion 10, which ensures the safe positioning of the protective cover 6 within the device body 5 during the injection operation. Only after the drug injection has been completely completed, the end, facing the protective cover 6, of the release ring 8 squeezes the claw 6.1 so that the claw 6.1 is detached from the snap-fitting portion 10, thereby enabling the protective cover 6 to be ejected relative to the device body 5. The protective cover 6 can be released and the safety device can be triggered without additional action, which is simple to operate and effectively prevents the protective cover from ejecting prematurely.

It should be noted that the elastic recovery force of the elastic member 7 is required to be greater than the force (the pushing force) for pushing the push rod 4 during the injection process. The elastic recovery force of the elastic member 7 may be determined based on the material and structure of the elastic member 7, and the pushing force of the pre-filled syringe 2 may be determined based on dimensions of the pre-filled syringe 2 (e.g. the size of the needle hole of the needle tip 11 and internal dimensions of the syringe body 13), which will not be specifically described here. The configuration would be applicable as long as the release ring 8 is positioned with reference to the device body 5 (to be specific, the protective cover 6) under the elastic recovery force of the elastic member 7 during the injection process. Therefore, the protective cover 6 can be released easily with one hand. In an embodiment, the claw 6.1 has a claw structure for outward grasping. After the claw 6.1 hooks the snap-fitting portion 10, there is a gap between the top of the claw 6.1 (the end, facing the release ring 8, of the claw 6.1) and the inner wall of the device body 5. When an end, facing the protective cover 6, of the release ring 8 squeezes the claw 6.1, the end of the release ring 8 extends into the gap and squeezes the claw 6.1 inwardly, so that the claw 6.1 moves away from the snap-fitting portion 10, thereby detaching the claw 6.1 from the snap-fitting portion 10. In combination with the elastic recovery force of the elastic member 7, the release ring 8 and the protective cover 6 move away from each other, thereby releasing the protective cover 6. Other structures may also be used, which will not be specifically described here.

In order to facilitate injection, an outer wall of the device body 5 is provided with an external flange 5.2. That is, during operation (including the injection process and the release of the protective cover), an operator holds the device body 5 and ensures that the device body 5 does not slide by positioning the external flange 5.2 against fingers, and then pushes the push rod 4 to complete the operation.

In order to facilitate assembly, the holder inner claw 9.2 is arranged at an end, away from the protective cover 6, of the holder 9. A holder outer claw 9.1 is arranged at another end, closer to the protective cover 6, of the holder 9. The device body 5 is provided with an opening portion 5.3. The holder outer claw 9.1 is fitted into the opening portion 5.3 for connection. In an embodiment, the holder outer claw 9.1 is of a structure sleeved inside the device body 5, the opening portion 5.3 is a through-hole, and the holder outer claw 9.1 hooks onto an inner wall of the through-hole to connect the holder 9 to the device body 5. The holder 9 may be connected with the device body 5 by means of other snap-fitting structures, which are not described here but shall fall within the scope of protection of the present application.

Further, a top projection ring 8.1 is provided at an end, away from the protective cover 6, of the release ring 8, and the positioning surface is a top surface of the top projection ring 8.1. An inner end face of the top projection ring 8.1 extends into an inner bore of the release ring 8, and an end of the elastic member 7 extends into the inner bore of the release ring 8 and abuts against the inner end face. With the above arrangement, the stability of the installation of the elastic member 7 is improved and the area of the positioning surface is increased, thereby ensuring stable contact between the syringe flange 13.1 and the top projection ring 8.1.

In an embodiment, the end, facing the protective cover 6, of the release ring 8 is a squeezing end for squeezing the claw 6.1. With the above arrangement, in the operation of releasing the protective cover 6, the syringe flange 13.1 pushes the release ring 8 toward the protective cover 6, and the end, facing the protective cover 6, of the release ring 8 squeezes the claw 6.1, so that the claw 6.1 is detached from the snap-fitting portion 10.

In an embodiment, the top projection ring 8.1 may further perform a positioning function, i.e., a stepped surface structure fitting with the top projection ring 8.1 is provided on the inner wall of the device body 5. Before or when the top projection ring 8.1 comes into contact with the stepped surface structure, the end (the squeezing end), facing the protective cover 6, of the release ring 8 squeezes the claw 6.1. With the above arrangement, in the operation of releasing the protective cover 6, the syringe flange 13.1 pushes the release ring 8 toward the protective cover 6. Before or when the top projection ring 8.1 comes into contact with the stepped surface structure, the end, facing the protective cover 6, of the release ring 8 has squeezed the claw 6.1, so that the claw 6.1 is detached from the snap-fitting portion 10.

In order to reduce the weight of the safety device, the holder 9 has a hollow structure. The provision of the hollow structure facilitates the elastic deformation of the holder 9 and the fitting and connection of the holder outer claw 9.1 to the opening portion 5.3.

Further, in order to improve the stability of the protective cover covering the needle tip 11 after the release of the protective cover 6, the bearing portion 5.1 is of a protruding ring structure with a first end face facing the release ring 8 and a second end face away from the release ring 8. A face, away from the release ring 8, of the positioning seat 6.4 is a limiting face. In the state that the needle tip 11 of the pre-filled syringe 2 is covered by the protective cover 6, the limiting face is in contact with the first end face for positioning. The effective contact between the limiting face and the first end face is ensured by the protruding ring structure of the bearing portion 5.1.

Further, the protective cover 6 is further provided with a positioning elastic sheet 6.3. In the state that the needle tip 11 of the pre-filled syringe 2 is covered by the protective cover 6, the positioning elastic sheet 6.3 is in contact with the second end face. It will be appreciated that the positioning elastic sheet 6.3 allows the protective cover 6 to move towards the needle tip 11, but restrains the protective cover 6 from moving towards the release ring 8. That is, after release of the protective cover 6, the bearing portion 5.1 is stuck between the positioning seat 6.4 and the positioning elastic sheet 6.3, further improving the stability of the protective cover covering the needle tip 11.

To facilitate the machining of components and parts, the elastic member 7 is a spring. The spring is mounted outside the pre-filled syringe 2 during installation. A rubber block or the like may be used as the elastic member 7, as long as it meets the requirements.

An injection device is further provided according to the present application. The injection device includes a pre-filled syringe 2 and the safety device according to any one of the above embodiments. As the above safety device has the technical effects described above, the injection device with the above safety device also has the same technical effects.

It should be emphasized that the above safety device can be adapted to common syringe structures without further improvements to the pre-filled syringe 2, as long as the size of the safety device is adjusted based on an actual size of the pre-filled syringe 2. Therefore, the safety device is highly versatile.

In an embodiment, the pre-fill syringe 2 may be used for pre-filling of the syringe body of 1.0 ml or 0.5 ml. In the pre-filled syringe 2 according to the embodiment of the present application, the size of the syringe body 13 is preferably 1/2 inch and the length of the pre-filling is slender to allow it to be wrapped by the safety device. The pre-filled syringe 2 is required to be provided with a push rod 4, so that the safety device can be effectively activated.

In an embodiment, the needle tip 11 of the pre-filled syringe 2 is covered with a needle housing 12, which is removed from the pre-filled syringe 2 prior to the injection operation. The syringe flange 13.1 is preferably arranged at a tail end of the syringe body 13.

The above front end refers to an end closer to the needle tip 11 and the tail end is an end away from the needle tip 11.

The safety device for a pre-filled syringe according to the present application is described in detail above. The principle and the embodiments of the present application are illustrated herein through specific examples. The description of the above-described embodiments is merely used to facilitate understanding the method and core idea of the present application. It should be noted that for those skilled in the art, various improvements and modifications can be made to the present application without departing from the principles of the present application, and these modifications and improvements are also deemed to fall into the scope of protection of the present application defined by the appended claims.

## Claims

1. A safety device for a pre-filled syringe, comprising:
- a device body (5), wherein an inner wall of the device body (5) is provided with a snap-fitting portion (10) and a bearing portion (5.1);
- a protective cover (6) movably arranged in the device body (5), wherein the protective cover (6) is provided with a claw (6.1) configured to fit with the snap-fitting portion (10) for positioning and a positioning seat (6.4) configured to restrain the protective cover (6) from escaping from the device body (5), wherein in a state that a needle tip (11) of the pre-filled syringe (2) is covered by the protective cover (6), the claw (6.1) is detached from the snap-fitting portion (10) and the positioning seat (6.4) is in contact with the bearing portion (5.1);
- a release ring (8) movably arranged in the device body (5), wherein an end, facing the protective cover (6), of the release ring (8) is configured to detach the claw (6.1) from the snap-fitting portion (10) by squeezing the claw (6.1), and another end, away from the protective cover (6), of the release ring (8) is a positioning surface configured to contact with a syringe flange (13.1) of the pre-filled syringe (2) for positioning;
- an elastic member (7), wherein an end of the elastic member (7) abuts against the release ring (8) and another end of the elastic member (7) abuts against the protective cover (6); and
- a holder (9) connected with the device body (5), wherein the holder (9) is provided with a holder inner claw (9.2) configured to restrain the syringe flange (13.1) from moving away from the release ring (8).

2. The safety device for a pre-filled syringe according to claim 1, wherein an outer wall of the device body (5) is provided with an external flange (5.2).

3. The safety device for a pre-filled syringe according to claim 1, wherein the holder inner claw (9.2) is arranged at an end, away from the protective cover (6), of the holder 9;
a holder outer claw (9.1) is arranged at another end, closer to the protective cover (6), of the holder (9), the device body (5) is provided with an opening portion (5.3), and the holder outer claw (9.1) is fitted into the opening portion (5.3) for connection.

4. The safety device for a pre-filled syringe according to claim 1, wherein a top projection ring (8.1) is provided at an end, away from the protective cover (6), of the release ring (8), and the positioning surface is a top surface of the top projection ring (8.1); an inner end face of the top projection ring (8.1) extends into an inner bore of the release ring (8), and an end of the elastic member (7) extends into the inner bore of the release ring (8) and abuts against the inner end face.

5. The safety device for a pre-filled syringe according to claim 1, wherein the end, facing the protective cover (6), of the release ring (8) is a squeezing end for squeezing the claw (6.1).

6. The safety device for a pre-filled syringe according to claim 1, wherein the holder (9) has a hollow structure.

7. The safety device for a pre-filled syringe according to claim 1, wherein the bearing portion (5.1) is of a protruding ring structure with a first end face facing the release ring (8) and a second end face away from the release ring (8); a face, away from the release ring (8), of the positioning seat (6.4) is a limiting face; and in the state that the needle tip (11) of the pre-filled syringe (2) is covered by the protective cover (6), the limiting face is in contact with the first end face for positioning.

8. The safety device for a pre-filled syringe according to claim 7, wherein the protective cover (6) is further provided with a positioning elastic sheet (6.3); and in the state that the needle tip (11) of the pre-filled syringe (2) is covered by the protective cover (6), the positioning elastic sheet (6.3) is in contact with the second end face.

9. The safety device for a pre-filled syringe according to any one of claims 1 to 8, wherein the elastic member (7) is a spring.

10. An injection device comprising a pre-filled syringe (2) and the safety device according to any one of claims 1 to 9.
